# EUROPEAN PATENT SPECIFICATION

(11) **EP 1 418 808 B1**
(45) Date of publication and mention of the grant of the patent: **30.11.2005**
(21) Application number: 02752606.0
(22) Date of filing: 29.07.2002
(51) Int. Cl.: A01K 67/033, A01K 67/027, G01N 33/00

(54) **IMAGING MARKER TRANSGENES**
ABBILDUNG VON TRANSGENEN MARKERN
TRANSGENES MARQUEURS D'IMAGERIE

(30) Priority: 30.07.2001 US 308919 P
(43) Date of publication of application: 19.05.2004
(73) Proprietor: SMITHKLINE BEECHAM CORPORATION, Philadelphia, PA 19101 (US)
(72) Inventor: NORTHROP, Jeffrey, Paul, Indianapolis, IN 46250 (US); STAR-LACK, Joshua, Morris, Palo Alto, CA 94303 (US)
(74) Representative: Nash, Guy Cameron
(86) International application number: PCT/US2002/023962
(87) International publication number: WO 2003/011020

(56) References cited:
- WO-A-01/57212
- WO-A-90/00625
- US-A- 5 185 142
- US-A- 5 256 395
- US-A- 6 017 754
- BERGER FRANK & GAMBHIR SANJIV: "recent advances in imaging endogenous or transferred gene expression utilizing radionuclide technologies in living subjects:applications to breast cancer" BREAST CANCER RESEARCH, vol. 3, 11 December 2000 (2000-12-11), pages 28-35, XP002289045

## Description

### BACKGROUND OF THE INVENTION

In recent years, mammalian genes have become amenable to experimental manipulation *in vivo;* due in large part to the isolation and characterization of genes, and to the ability to manipulate an embryo's genotype through transgenic animal technology. The increase in sequence information has made it possible at the molecular level to introduce a targeted mutation into virtually any sequence through gene targeting in totipotent embryonic stem (ES) cells. The ability to knock out either a specific gene or a specific cell type in an intact animal offers great opportunities for addressing questions concerning molecular and cellular biology. As well, animal models for various human diseases can be generated; including the introduction of genetically altered human cells into animals.

A key point in creating and studying these introduced genetic changes is the ability to "mark" the genetically altered cells, and to be able to follow patterns of gene expression in response to stimuli. Three aspects of gene therapy are of particular interest to imaging. The first is in delivering genes and vector products by minimally invasive interventional techniques. The second is in quantitating gene and DNA deliveries, for example, by nuclear imaging. Finally, imaging can be used to monitor the levels of transgene expression *in vivo* (Wunderbaldinger *et al*. (2000) Eur. J. Radiol. **34**:156-165). Approaches using the herpes simplex type 1 virus thymidine kinase (HSV1-tk) and the dopamine type 2 receptor (D2R) genes in conjunction with radiolabeled probes have been described (see, for example Gambhir *et al*. (2000) Neoplasia **2**:118-138; Blasberg *et al*. (1999) Q J Nucl Med **43**(2):163-9). The HSV1-tk gene acts by reacting with a radiolabeled substrate, for example 5-iodo-2'-fluoro-2'deoxy-1-beta-D-arabino-furanosyl-uracil (FIAU), ganciclovir and acyclovir. The magnitude of FIAU accumulation is correlated with HSV1-tk expression.

One approach to non-invasive imaging utilizes three-dimensional (3-D) emission computed tomography (ECT), which provides a qualitative and quantitative look at the volume distribution of biologically significant radiotracers after injection into the body. Emission computed tomography, together with two-dimensional (2-D) planar imaging, is the main imaging techniques used in nuclear medicine. PET and SPECT are imaging techniques in which a radionuclide is synthetically introduced into a molecule or ligand of potential biological relevance and administered to a patient. Depending on the nature of the radiopharmaceutical, it may be inhaled, ingested, or, most commonly, injected intravenously. The subsequent uptake of the radiotracer is measured over time and used to obtain information about the physiological process of interest. Because of the high-energy (γ-ray) emissions of the specific isotopes employed and the sensitivity and sophistication of the instruments used to detect them, the two-dimensional distribution of radioactivity may be inferred from outside of the body.

Improved methods of detecting *in vivo* changes in gene expression, particularly non-invasive techniques for following changes in gene expression, are of great interest. Animal studies geared to monitoring the up- and down-regulation of a given gene in response to a specific challenge will be extremely valuable in screening candidate drugs, as well as in identifying and validating new targets.

### Relevant Literature

Receptor mediated targeting of fluorescent probes in living cells is described in Farinas and Werkman (1999) J. Biol. Chem. **274**:7603-7606. Power and Hudson (2000) J. Immunol. Meth. 242:193-204 review the synthesis of high avidity antibody fragments for cancer imaging. Vaughan et al. (1996) Nat. Biotech. 14:309-314 describe methods of screening phage display libraries for high affinity antibody binding.

Berger & Gambhir, (2000), Breast Cancer Res., 3, 28-35 describes various in vivo methods for detecting gene expression.

### SUMMARY OF THE INVENTION

Non human Transgenic animals and cells comprising an imaging marker transgene are provided. The imaging marker is expressed on the cell surface, and is capable of binding at high affinity a radiolabeled hapten that provides a signal useful in emission computed tomography. A preferred imaging marker is an antibody fragment, *e.g.* a single chain variable region (scFv). Radiolabeled haptens of interest include metal chelates, and may also include small molecules suitable for radiolabeling, e.g. digoxigenin, substituted triazines, and the like. The animals or cells are monitored *in* *vivo* by administering the radiolabeled hapten, and localizing expression through SPECT or PET acquisition and reconstruction.

The transgene may be operably linked to a regulated promoter of interest, where the level of expression of the transgene provides information about the promoter. Alternatively, the imaging marker transgene is controlled by a constitutive promoter, and serves as a marker for the presence of the genetically modified cells. The animals and cells find use in screening compounds for their physiological effectiveness *in vivo*. The imaging markers also find use in tracing the presence of a transgene, which may be selectively present on a cell type of interest, *e.g.* tumor cells, transplanted cells, *etc.*

### BRIEF DESCRIPTION OF THE DRAWINGS

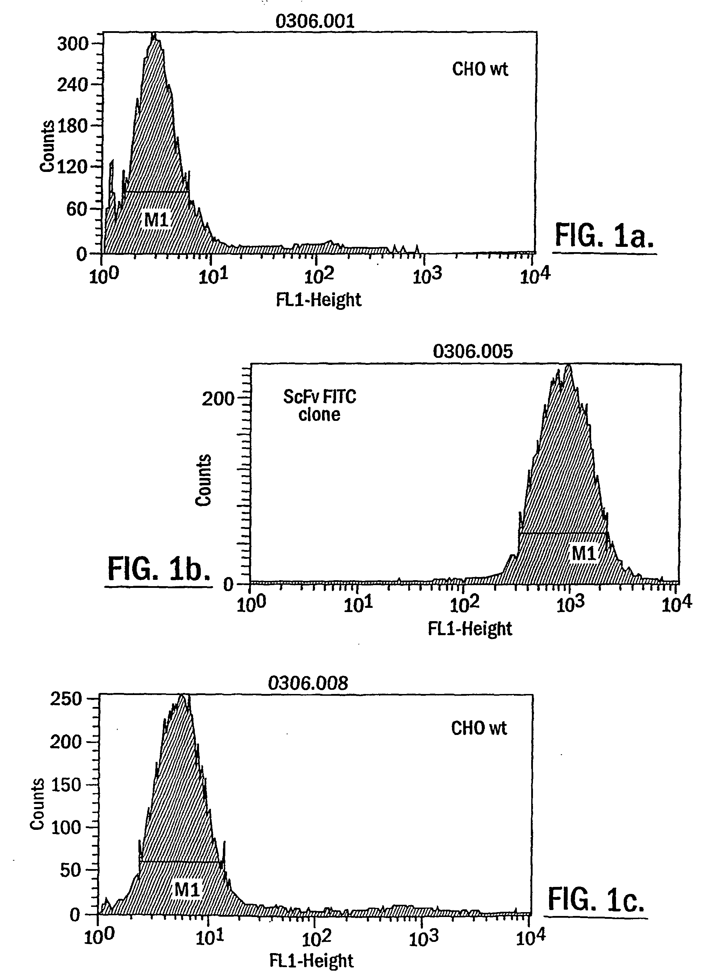

Figures 1A - 1D: Single chain antibody (ScFv) expression in CHO cells. Wild-type CHO cells (CHO wt) or a clone expressing the FITC ScFv were stained with either anti-179 antibody linked to FITC (left) or with BSA linked to FITC (right). The FITC ScFv expression construct contains the epitope recognized by the 179 antibody. Cells were analyzed by FACS. The mean fluorescence of ScFv-FITC expressing cells is 298-fold higher than CHO wt cells with 179 stain and 23-fold higher with the BSA stain. The BSA-FITC reagent can only be captured on the cell surface through the FITC molecule being bound by the ScFv, while the 179-FITC reagent could be bound through the 179 epitope or through binding of the FITC.

**Figure 1A:**

| Wild-type CHO cells stained with anti-179 antibody. | | | | |
|---|---|---|---|---|
| Marker | Left, Right | Events | Mean | Median |
| All | 1,9910 | 31990 | 5.22 | 2.97 |
| M1 | 1, 6 | 28143 | **3.09** | 2.89 |

**Figure 1B:**

| Cells expressing FITC ScFv stained with anti-179 antibody. | | | | |
|---|---|---|---|---|
| Marker | Left, Right | Events | Mean | Median |
| All | 1,9910 | 32260 | 943.92 | 820.47 |
| M1 | 331,2267 | 30497 | **922.36** | 827.88 |

**Figure 1C:**

| Wild-type CHO cells stained with BSA. | | | | |
|---|---|---|---|---|
| Marker | Left, Right | Events | Mean | Median |
| All | 1,9910 | 30862 | 17.26 | 5.33 |
| M1 | 2, 14 | 28246 | **5.76** | 5.23 |

**Figure 1D:**

| Cells expressing FITC ScFv stained with BSA. | | | | |
|---|---|---|---|---|
| Marker | Left, Right | Events | Mean | Median |
| All | 1,9910 | 32068 | 141.78 | 124.09 |
| M1 | 63, 257 | 29089 | **131.14** | 125.21 |

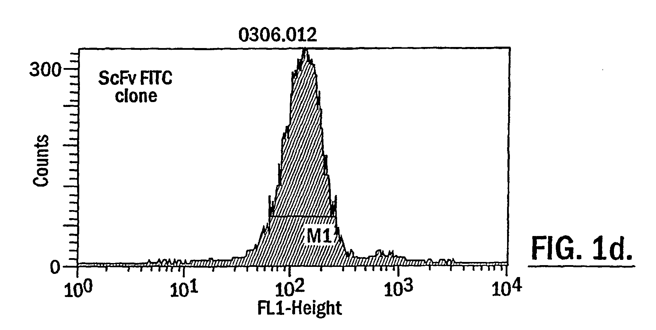

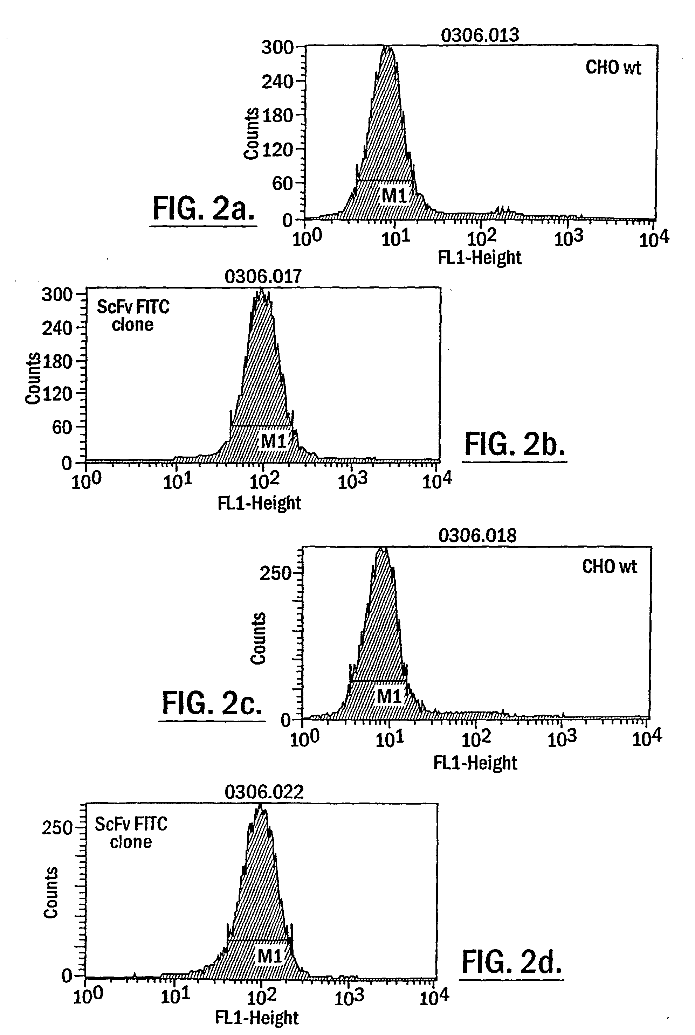

Figure 2A - 2D: Staining of CHO cells with FITC coupled to the metal chelate, DPTA. Same cells as in Figure 1, but stained with DTPA-FITC or Indium DTPA-FITC followed by FACS analysis. In each case, the ScFv-FITC-expressing cells are 11- to 12-fold brighter than CHO wt cells indicating that indium does not alter binding of the ScFv to FITC.

**Figure 2A:**

| Wild-type CHO cells stained with DTPA-FITC. | | | | |
|---|---|---|---|---|
| Marker | Left, Right | Events | Mean | Median |
| All | 1,9910 | 31969 | 13.89 | 7.99 |
| M1 | 4, 17 | 28507 | **8.24** | 7.84 |

**Figure 2B:**

| Cells expressing FITC ScFv stained with DTPA-FITC. | | | | |
|---|---|---|---|---|
| Marker | Left, Right | Events | Mean | Median |
| All | 1,9910 | 31930 | 100.62 | 90.58 |
| M1 | 42, 213 | 29885 | **98.06** | 91.40 |

**Figure 2C:**

| Wild-type CHO cells stained with Indium DTPA-FITC. | | | | |
|---|---|---|---|---|
| Marker | Left, Right | Events | Mean | Median |
| All | 1, 9910 | 30783 | 11.54 | 7.77 |
| M1 | 3, 15 | 27649 | 7.87 | 7.50 |

**Figure 2D:**

| Cells expressing FITC ScFv stained with Indium DTPA-FITC. | | | | |
|---|---|---|---|---|
| Marker | Left, Right | Events | Mean | Median |
| All | 1, 9910 | 31167 | 95.46 | 87.38 |
| M1 | 40, 225 | 29163 | **96.09** | 88.96 |

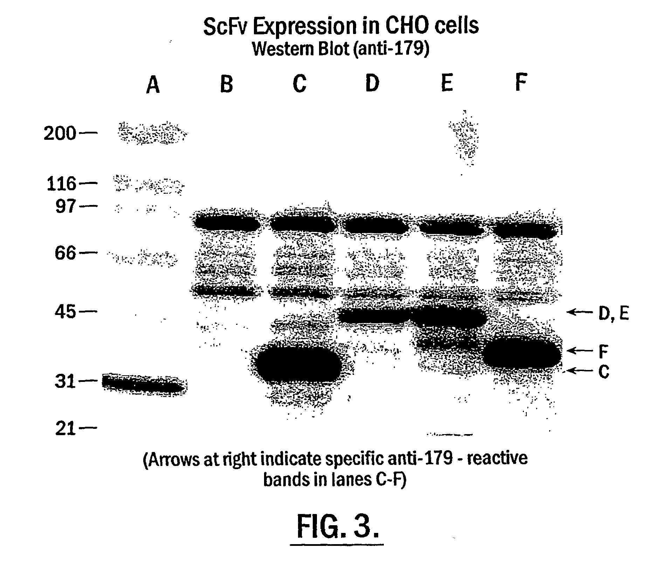

Figure 3: Expression of GPI-linked or transmembrane-linked ScFVs in CHO cells. Analysis of whole cell lysates by anti-179 western blotting. Lane A = molecular weight markers (kilodaltons); Lane B = mock (CHO wt); Lane C = ScFv FITC-GPI linkage; Lane D = ScFv FITC - CD8-zeta fusion; Lane E = ScFv FITC - CD8-zeta fusion; Lane F = ScFv FITC - CD8TM fusion. Lanes C through F show that the ScFv to FITC can be expressed as a GPI-linked protein (as in Figures 1 and 2) or as a transmembrane protein (CD8 TM used) with either little to no intracytoplasmic domain, or with a significant intracytoplasmic domain (the T-cell receptor zeta chain).

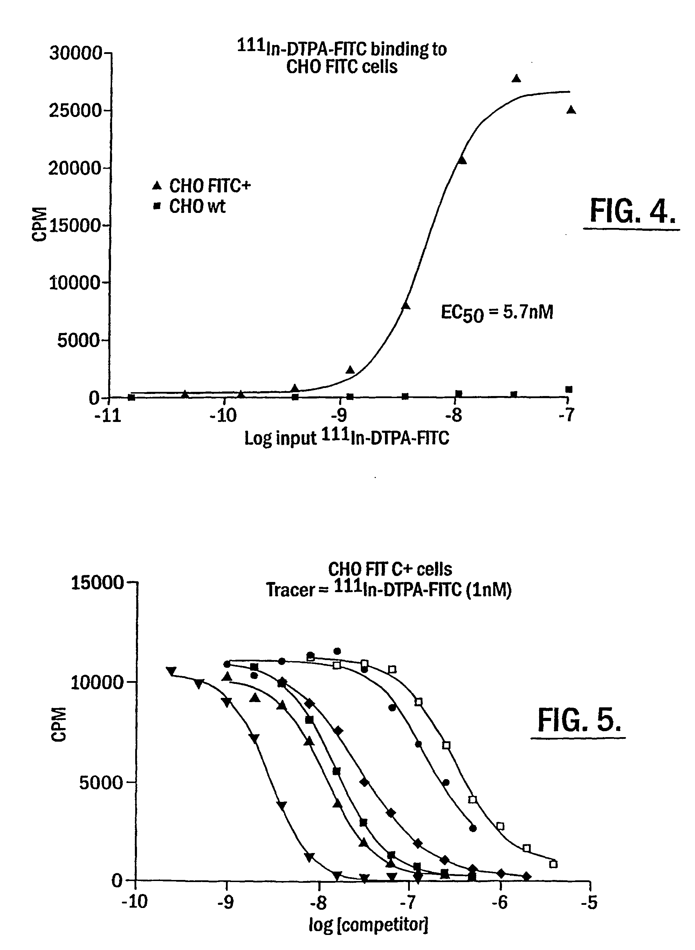

Figure 4: Binding of 111In-DTPA-FITC to CHO cells. CHO wild-type (wt) or ScFv-expressing (FITC+) cells incubated with varying concentrations of 111In-DTPA-FITC. The concentration to half-maximally saturate (EC₅₀) the ScFv is 5.7nM. wild-type cells do not bind the probe. Data expressed as counts per minute (CPM) bound.
Figure 5: Competition binding on CHO ScFv FITC-expressing cells. Four FITC derivates, fluorescein or erythrosine were added at varying concentrations in the presence of 1nM 111In-DTPA-FITC as tracer. (In)-DTPA-FITC has an IC₅₀ value of 11-15nM while the multivalent competitor, FITC-BSA, is more potent and unmodified fluorescein and its iodinated derivative, erythrosine, are less potent. IN-FITC-DTPA (IC₅₀ 15.0 nM) indicated by (■); FITC-DTPA (IC₅₀ 11.8 nM) indicated by (▲); FITC-BSA (IC₅₀ 2.9 nM) indicated by (▼); fluorescein (IC₅₀ 27.1 nM) indicated by (◆); FITC-isoamylamine (IC₅₀ 151 nM) indicated by (●); erythrosine (IC₅₀ 309 nM) indicated by (□).

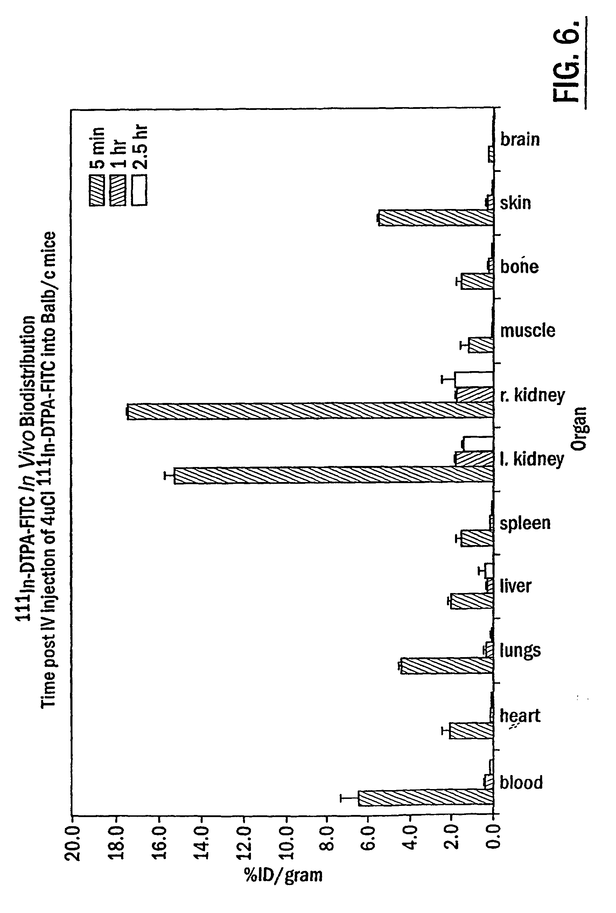

Figure 6: *In vivo* biodistribution of 111In-DTPA-FITC. Duplicate female Balb/c mice were injected IV by tail vein with 4uCi 111In-DTPA-FITC and sacrificed at the indicated times (5 minutes, 1 hour, 2.5 hours). Tissue samples were then weighed and counted in a gamma counter. Data is displayed as % injected dose per gram of tissue. The tracer appears to be rapidly cleared by the kidneys.

### DESCRIPTION OF THE SPECIFIC EMBODIMENTS

An imaging marker system is provided to uniquely identify cells that contain a transgene. Cells comprising the transgene may be isolated and cultured *in vitro* for analysis of gene expression in different cell populations, or may be present in an in vivo system, e.g. a non human transgenic animal comprising the transgene, or a chimeric animal where a subset of cells comprise the transgene. A chimeric animal may comprise, e.g. transgenic tumor cells, transplants such as blood, kidney, liver, etc., and the like. The animals or cells are monitored *in vivo* by administering a radiolabeled imaging agent, and then localizing expression through SPECT or PET acquisition and reconstruction.

A suitable imaging marker is expressed on the cell surface, and is capable of binding at high affinity a radiolabeled hapten. A preferred imaging marker is an antibody fragment, e.g. a single chain variable region (scFv). Radiolabeled haptens of interest include metal chelates, such Indium DTPA, *etc.,* and may also include small molecules suitable for radiolabeling, e.g. digoxigenin, substituted triazines, and the like.

The term "transgene" is used herein to describe genetic material that has been or is about to be artificially inserted into the genome of an animal cell, particularly a cell of a living animal. The transgene is used to transform a cell, meaning that a permanent or transient genetic change, preferably a permanent genetic change, is induced in a cell following incorporation of exogenous DNA. A permanent genetic change is generally achieved by introduction of the DNA into the genome of the cell.

The transgene may be operably linked to a regulated promoter of interest, where the level of expression of the transgene provides information about the promoter. Optionally, the transgene is also operably linked to a transgene of interest, which can be linked to the same or a different promoter. Alternatively, the imaging marker transgene is controlled by a constitutive promoter, and serves as a marker for the presence of the genetically modified cells. The animals and cells find use in screening compounds for their physiological effectiveness *in vivo.* The imaging markers also find use in tracing the presence of a transgene, which may be selectively present on a cell type of interest, e.g. tumor cells, transplanted cells, *etc.*

### IMAGING MARKER GENE.

Imaging marker genes for use in the present invention have the characteristics of being expressed on the cell surface, and binding at high affinity a hapten that can be radiolabeled. Generally, the imaging marker gene is not normally expressed in the cells into which it is introduced, nor on any cell normally present in a genetically unmodified animal. Suitable imaging marker genes include, for example avidin, which binds at very high affinity to biotin and biotinylated haptens; immunological binding domains, *e.g*. T cell receptor, antibodies, etc. A preferred imaging marker gene is a single chain variable fragment from an antibody.

Reference to "antibody" or "antibody fragment" means a polypeptide member which has its binding site formed by association of a first polypeptide domain that comprises a binding region of an immunoglobulin heavy chain variable domain (VH) and a second polypeptide domain that comprises a binding region of an immunoglobulin light chain variable domain (VL). Single chain Fv (scFv) comprises a VL domain and a VH domain linked by a peptide linker.

A single chain Fv polypeptide is a covalently linked V_{H}V_{L} heterodimer, which may be expressed from a nucleic acid including V_{H} - and V_{L} -encoding sequences either joined directly or joined by a peptide-encoding linker (see Bird *et al*. (1988) Science **242**:423-426; Huston *et al*. (1988) P.N.A.S. **85**: 5879-5883; and Vaughan *et al*., *supra*.) A number of structures are known in the art for converting the naturally aggregated but chemically separated light and heavy polypeptide chains from an antibody V region into an sFv molecule which will fold into a three dimensional structure substantially similar to the structure of an antigen-binding site. See, *e.g*. U.S. Pat. Nos. 5,091,513 and 5,132,405 and 4,956,778.

Design criteria include determination of the appropriate length to span the distance between the C-terminal of one chain and the N-terminal of the other, wherein the linker is generally formed from small hydrophilic amino acid residues that do not tend to coil or form secondary structures. Such methods have been described in the art. Appropriate linkage sites on each of the V_{H} and V_{L} polypeptide domains include those that will result in the minimum loss of residues from the polypeptide domains, and a linker comprising a minimum number of residues consistent with the need for molecule stability. Thus, suitable linkers generally comprise polypeptide chains of alternating sets of glycine and serine residues, and may include glutamic acid and lysine residues inserted to enhance solubility. Nucleotide sequences encoding such linker moieties can be readily provided using various oligonucleotide synthesis techniques known in the art.

The phrase "specifically binds to a hapten", when referring to an antibody, refers to a binding reaction which is determinative of the presence of the hapten in the presence of a complex mixture, e.g. in the blood stream or extravascular space of an animal. A variety of immunoassay formats may be used to select antibodies specifically immunoreactive with a particular hapten. For example, phage display is routinely used in the screening of scFv binding, or solid-phase ELISA immunoassays in a variety of formats are known in the art. See Harlow and Lane (1988) Antibodies, A Laboratory Manual, Cold Spring Harbor Publications, New York, for a description of immunoassay formats and conditions that can be used to determine specific immunoreactivity.

In order to prepare recombinant antibody fragments from hybridomas, the genetic information which encodes the antigen-binding domains (VH and VL) of the antibodies is obtained by isolating the mRNA, reverse-transcribing the RNA into cDNA and then amplifying the cDNA by cloning, or by means of the polymerase chain reaction and using oligonucleotides that are complementary to the 5' and 3' ends of the variable fragments. The VH and VL fragments are then cloned into expression vectors].

The phage-display technique can also be used to isolate new antibody fragments directly from antibody libraries of, for example, murine or human origin. In the phage-display of antibody fragments, the antigen-binding domains are cloned as protein fusions with the coat protein g3P of filamentous bacteriophages, either into the phage genome or into phagemid vectors in the form of scFv fragments (see McCafferty *et al*. (1990) Nature **348:**552). Antigen-binding phages are selected on antigen-loaded plastic vessels (panning), on antigen-conjugated, paramagnetic beads, by binding to cell surfaces, and the like.

### MEMBRANE LOCALIZATION

The imaging marker polypeptides can be anchored to cell membranes through hydrophobic membrane spanning domains (transmembrane domains) as well as through post-translational attachment of the covalently linked glycosylated form of phosphatidylinositol (GPI membrane anchor). Sequences signaling GPI linkage causes the chimeric GPI-anchored imaging marker to be membrane bound (Wettstein *et al*. (1991) J. Exp. Med. **174**:219-228). The imaging marker gene can thus be fused to appropriate signal sequences directing GPI anchoring. GPI-linked membrane anchor domains have been defined in a number of proteins including decay accelerating factor (DAF), CD59 and humans placental alkaline phosphatase (HPAP). For example, the 38 carboxyl terminal amino acids of HPAP are sufficient to act as a signal sequence for GPI linkage. The imaging marker polypeptide becomes attached through its carboxy terminus to the extracellular surface through an oligosaccharide bound to an inositol phospholipid. The proteins can be cleaved from the surface using a phosphatidylinositol-specific phospholipase C, and the spacing of the protein from the membrane can be varied. In addition, the proteins can easily be expressed with or without added antibody epitopes.

Alternatively, a transmembrane domain may be fused to the imaging marker gene. The term "transmembrane domain" refers to the domain of the protein that crosses the plasma membrane, and is derived from an antibody gene, or is a domain associated with a totally different protein. The sequences of many transmembrane domains are known and available from public databases. Alternatively, the transmembrane domain may be an artificial hydrophobic amino acid sequence that spans the plasma cell membrane.

When the imaging marker utilizes a transmembrane domain for cell surface localization, it may further comprise a cytoplasmic domain, which can serve a variety of purposes. For example, an intracellular signaling domain can be provided, which is then activated upon binding to the hapten. This has advantages with respect to receptor internalization or turnover.

### HAPTENS AND LABELS

The hapten specific imaging marker will usually have an affinity of at least about 100 nM for the hapten. Suitable haptens of interest include metal chelates, *e.g.* ¹¹¹I-DTPA; ^{99m}Tc-DTPA; ⁶⁷Ga-EDTA; ⁶⁸Ga-EDTA; DOTA complexes; *etc.,* or haptens conjugated to a label, *e.g*. digoxin, digoxigenin, FITC, dinitrophenyl, nitrophenyl, biotin, *etc*. For example, haptens may include such molecules as endogenous hormones, drugs, herbicides, fluorescent compounds, PCBs and aromatics. Specific haptens of interest can be easily modified with metal chelates or positron emitters, *e.g*. tri-substituted triazines, where R1 comprises the hapten epitope, and R2 and R3 are building blocks for positron emitter, biodistribution modifier, metal chelate, *etc*. Methods for conjugation of haptens to a label are known in the art.

Suitable labels of the invention are those that can serve for PET or SPECT imaging. For example ¹¹¹In; ^{99m}Tc; ⁶⁷Ga; ⁶⁸Ga; ¹⁸F; ¹¹C; ¹⁵O, Br isotopes; *etc*. For both PET and SPECT, specific activities of greater than 2000 Ci/mmol are generally desirable. The particularly narrow radiochemical window for positron-emitting radionuclides has special implications PET-imaging. Specifically, the most commonly used PET isotopes include ¹⁵O, ¹³N, ¹¹C, and ¹⁸F, which have half-lives of 2, 10, 20, and 109 min, respectively. Because of their extremely rapid decay, essentially all PET isotopes are produced on site. In contrast, SPECT isotopes, like ¹²³I, have a sufficiently long half-life to enable centralized production at distant commercial reactors and to allow delivery via express mail. Alternatively, ^{99m}Tc may be obtained from inexpensive molybdenum generators located in most hospital radiopharmacies.

For example, ¹¹C can be relatively easily substituted for ¹²C in existing organic molecules without altering their chemical properties. Even though fluorine is not commonly found in human biochemistry, native hydrogen moieties may frequently be replaced by ¹⁸F without significant isotopic effects. In contrast, SPECT nuclides are not intrinsic to most biological substrates. The metallic nature and multiple valence states of, for example, ^{99m}Tc, generally necessitates complexing groups for its molecular stabilization. Iodinated radioligands and other halogen radiolabels, *e.g.* ¹⁸F; ¹²³I; ¹³³Xe; *etc.* find use where the biochemical or physiological activities of any halogenated compound can generally be substituted with a radiolabel.

### Vectors

The genetic sequences encoding the imaging marker are introduced into an expression vector. Methods that are well known to those skilled in the art can be used to construct expression vectors containing coding sequences and appropriate transcriptional and translational control signals for increased expression of an exogenous gene introduced into a cell. These methods include, for example, *in vitro* recombinant DNA techniques, synthetic techniques, and *in vivo* genetic recombination. Alternatively, RNA capable of encoding gene product sequences may be chemically synthesized using, for example, synthesizers. See, for example, the techniques described in "Oligonucleotide Synthesis", 1984, Gait, M. J. ed., IRL Press, Oxford. In one embodiment of the invention, the vector construct will coordinately express a test gene and the imaging marker gene, such that expression of the imaging marker gene can be used as an indicator for the expression of the test gene, as well as for analysis of gene transfer efficiency.

A variety of host-expression vector systems can be utilized to express the imaging marker gene. In order to integrate into the chromosome of an animal cell, the construct will include regions of homology to the targeted region of the chromosome. The exact placement of such homologous regions determines whether the construct replaces chromosomal sequences, or inserts into the chromosome. The regions of homology to the chromosome, herein termed targeting sequences, flank the heterologous recombination site, repressor, marker, promoters, etc. The targeting sequences will preferably comprise at least about 50 nucleotides of nucleotide sequence, more usually at least about 100 nt of sequence, and may comprise 1000 nt of sequence, or more. The selection of targeting sequences will determine where in the genome the marker system will be integrated. There is substantial sequence information available for the genomes of mice, rats, *etc.,* and therefore one can easily select a region of the genome for targeting.

In mammalian host cells, a number of viral-based expression systems may also be utilized, *e.g*. retroviral vector derived from MoMLV, MSCV, SFFV, MPSV, SNV, *etc*., lentiviral vectors derived from HTV-1, HIV-2, SIV, BIV, FIV *etc*.; adeno-associated virus (AAV) vectors, adenoviral vectors derived from Ad5 virus, *etc*.; SV40-based vectors; Herpes Simplex Virus (HSV)-based vectors; *etc*.

In cases where an adenovirus is used as an expression vector, the imaging marker sequence may be ligated to an adenovirus transcription/translation control complex, *e*.*g*., the late promoter and tripartite leader sequence. This chimeric gene may then be inserted in the adenovirus genome by *in vitro* or *in* vivo recombination. Insertion in a non-essential region of the viral genome (*e*.*g*., region El or E3) will result in a recombinant virus that is viable and capable of expressing the gene product in infected hosts (*see* Logan & Shenk, 1984, Proc. Natl. Acad. Sci. USA 81:3655-3659). Specific initiation signals may also be required for efficient translation of inserted gene product coding sequences. These signals include the ATG initiation codon and adjacent sequences. Standard systems for generating adenoviral vectors for expression on inserted sequences are available from commercial sources, for example the Adeno-X™ expression system from Clontech (Clontechniques (January 2000) p. 10-12).

Exogenous translational control signals, which may include the ATG initiation codon, may also be provided. The initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, *etc.* (see Bittner *et al*., 1987, Methods in Enzymol. 153:516-544).

### PROMOTERS

Various promoters can be used to control expression of the imaging marker. The promoter used will vary with the intended aim of the method of the invention. For example, where the method is to be used to track the presence of transfected cells, a constitutive promoter can be used, or one that is externally regulated, *e*.*g*. a promoter from cytomegalovirus (CMV), mouse mammary tumor virus (MMTV), Rous sarcoma virus (RSV), or adenovirus. More specifically, exemplary promoters include the promoter from the immediate early gene of human CMV (Boshart et al., Cell 41:521-530, 1985; promoter from the long terminal repeat (LTR) of RSV (Gorman et al., Proc. Natl. Acad. Sci. USA 79:6777-6781, 1982); metallothionein promoter, elongation factor promoter, actin promoter, *etc*., from mammalian viruses, *e.g.,* the adenovirus late promoter; the vaccinia virus 7.5K promoter, SV40 late promoter, cytomegalovirus, *etc.*

Alternatively, the imaging marker can be used to study the effect of a treatment, drug, *etc.* on expression from a particular promoter. The promoter used may be regulated by a pathway of interest, *e.g.* by the presence of a signaling molecule; tissue-specific; cell type-specific promoter; *etc*. For example, the promoter can be one designed for substantially specific expression within a specific tissue. Exemplary tissue-specific or cell-specific promoters include, but are not limited to, myosin heavy chain promoter for muscle specific expression, Madsen *et al*. (1998) Circ Res 82(8):908-917; lysosomal acid lipase promoter, Du *et al*. (1998) Gene 208(2):285-295; pancreatic expression using the amylase promoter, Dematteo et al. (1997) J Surg Res72(2):155-161; cardiac-specific overexpression, Kubota et al. (1997) Circ Res 81(4):627-635; folylpoly-gamma-glutamate synthetase promoter, Freemantle et al. (1997) J Biol Chem 272(40):25373-25379; tissue specific expression using neural restrictive silencer element, Kallunki et al. (1997) J Cell Biol 138(6):1343-1354 , placenta specific expression using the HGH promoter, Nogues et al. (1997) Endocrinology 138(8):3222-3227, expression during pregnancy using the prolactin promoter, Schuler et al. (1997) Endocrinology 138(8):3187-3194, tissue specific expression using the alphal(VI) collagen promoter, Braghetta et al. (1997) Eur J Biochem 247(1):200-208; B cell specific expression, Lennon et al. (1997) Immunogenetics 45(4):266-273; hypoxia induced expression, Gupta et al. (1996) Nucleic Acids Res 24(23):4768-4774; endothelium specific expression, Ronicke et al. (1996) Circ Res 79(2):277-285, the keratin promoters (e.g., human keratin 14 promoter (Wang et al. 1997 Proc Natl Acad Sci US 94:219-26); bovine cytokeratin gene promoters, BKIII and BKVI (Alexander et al. 1995 Hum Mol Genet 4:993-9); keratin 10 gene promoter (Bailleul et al. 1990 Cell 62:697-708); and tyrosinase promoters (specific for melanocytes)). Epidermal-specific promoters are reviewed in Fuchs et al. 1994 Princess Takamatsu Symp 24:290-302).

Inclusion of an Internal Ribosome Entry Site (IRES), allows the marker or reporter gene to be coordinately expressed with another transgene by linking the test and a marker gene with an internal ribosomal entry site (IRES) sequence and expressing both genes from a single bi-cistronic mRNA. Where normally a single protein will be translated from an mRNA, the addition of an IRES sequence allows additional translational starts. An example of a retroviral construct containing IRES sequences is described in Zitvogel *et al*. (1994) Hum. Gene Ther. **5**:1493-1506. IRES sequence could be from a virus (e.g. EMCV, FMDV etc) or a cellular gene (e.g. eIF4G, BiP, Kv1.4 etc).

### NON HUMAN TRANSGENIC CELLS AND ANIMALS

Non human Transgenic animals comprise an exogenous nucleic acid sequence present as an extrachromosomal element or stably integrated in all or a portion of its cells, especially in germ cells. Unless otherwise indicated, it will be assumed that a transgenic animal comprises stable changes to the germline sequence. During the initial construction of the animal, "chimeras" or "chimeric animals" are generated, in which only a subset of cells have the altered genome. Chimeras are primarily used for breeding purposes in order to generate the desired transgenic animal. Animals having a heterozygous alteration are generated by breeding of chimeras. Male and female heterozygotes are typically bred to generate homozygous animals. Of interest are transgenic mammals, *e.g.* cows, pigs, goats, horses, *etc.,* and particularly rodents, *e.g.* rats, mice, *etc.*

The vector comprising the imaging marker gene can be used to transform an non human ES cell. For non human embryonic stem (ES) cells, an non human ES cell line may be employed, or non human embryonic cells may be obtained freshly from a host, *e.g*. mouse, rat, guinea pig, *etc.* Such cells are grown on an appropriate fibroblast-feeder layer or grown in the presence of leukemia inhibiting factor (LIF). When non human ES or embryonic cells have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the construct. Those colonies that are positive may then be used for embryo manipulation and blastocyst injection.

Blastocysts may be obtained from 4 to 6 week old superovulated females by flushing the uterus 3.5 days after ovulation. The embryonic stem cells are then trypsinized and the modified cells added to a droplet containing the blastocysts. At least one, usually at least about 10, and up to about 30 of the modified embryonic stem cells may be injected into the blastocoel of the blastocyst. After injection, at least one and not more than about 15 of the blastocysts are returned to each uterine horn of pseudopregnant females. Females are then allowed to go to term and the resulting litters screened for cells having the construct. The blastocysts are usually chosen to have a different parentage from the transformed ES cells. By providing for a different phenotype of the blastocyst and the ES cells, chimeric progeny can be readily detected. A particularly useful phenotype is hair color, although any phenotype or genotype may be used.

The pups will usually be born 16-18 days after introduction of the blastocysts into foster mothers. The chimeric animals are screened for the presence of the integrated construct at one locus (heterozygotes). Male and female heterozygotes are mated to generate homozygotes. If the gene alterations cause lethality at some point in development, heterozygous strains will be maintained as breeding stock.

### COMPUTED EMISSION TOMOGRAPHY

The imaging marker and radiolabeled hapten of the invention are used in conjunction with imaging techniques, in order to analyze the expression of the imaging marker, for example when the marker is operably linked to a regulated promoter, or as a tracer for transplanted cells, tumor xenografts, and the like. In a preferred embodiment, the imaging method is one of PET or SPECT, which are imaging techniques in which a radionuclide is synthetically introduced into the hapten and administered to an animal. Depending on the nature of the so-called radiolabeled hapten, it may be inhaled, ingested, or, most commonly, injected intravenously. The subsequent uptake of the radiotracer is measured over time and used to obtain information about the physiological process of interest. Because of the high-energy (γ-ray) emissions of the specific isotopes employed and the sensitivity and sophistication of the instruments used to detect them, the two-dimensional distribution of radioactivity may be inferred from outside of the body.

Radionuclides useful in PET imaging decay by converting a proton to a neutron and positron. A positron encountering an electron is annihilated, resulting the production of two γ photons, each of equivalent energy and opposite trajectory. Among the most commonly used positron-emitting nuclides in PET are included ¹¹C, ¹³N, ¹⁵O, and ¹⁸F. The unique spatial signature of back-to-back photon paths is exploited by PET scanners in locating the source of an annihilation event, a method known as coincidence detection. PET and SPECT scanners employ highly sensitive scintillation detectors made of dense crystalline materials that capture the high-energy □ rays and convert them to visible light. This brief flash of light is converted into an electrical pulse by an immediately adjacent photomultiplier tube (PMT). The crystal and PMT together make up a radiation detector. Rather than using individual detectors in isolation, a PET camera is constructed such that opposing detectors are electronically connected. Thus, when separate scintillation events in paired detectors coincide, an annihilation event is presumed to have occurred at some point along an imaginary line between the two. This information is registered by a computer and later used to reconstruct images using the principles of computed tomography.

Alternatively, radionuclides may instead capture an orbiting electron, transforming a proton to a neutron. This metastable arrangement subsequently dissipates, producing a single γ photon in the process. Isotopes that decay by electron capture and/or γ emission are used in SPECT, and include ¹²³I and ^{99m}Tc. The emission of a single photon means that instrumentation in SPECT must be intrinsically different from that in PET. Instead of coincidence detection, SPECT utilizes collimation.

Although the physical principles relating to photon emission and detection are different, the means by which PET and SPECT translate information about photon paths into cross-sectional images are largely the same. Because only information about a photon's direction, not depth, is known, views of photon trajectories from multiple angles are required. A set of measurements from a given angle or viewpoint is referred to as a projection. In PET, multiple projections are obtained by a ring of essentially contiguous radiation detectors, whereas SPECT cameras typically use several detector heads that rotate around the subject in synchrony and collect data over an entire 360°. After recording trajectories from multiple projections, a picture of the distribution of radioactivity within a given tissue slice is created. Typically, a modified technique known as filtered backprojection is applied.

### METHODS OF ANALYSIS

Non human Animals comprising the imaging marker transgene of the invention are useful in a variety of screening assays. In one aspect of the invention, cells comprising the imaging marker are transplanted into a host animal, and imaging techniques are used to track the presence, growth and/or responsiveness of the cells to stimulus. In another aspect, the imaging marker is provided with a genetic therapy vector, where the presence of the imaging marker is used to determine the presence of transfected cells.

Alternatively, the transgene is present in a non human transgenic animal, which is then subjected to a stimulus of interest, for example a test compound, therapeutic intervention, etc., and the response of a particular promoter of interest is monitored through expression of the transgene. A test cell is exposed to at least one candidate substance or treatment; and it is then detected whether such exposure induces expression of the imaging marker that is in operative association with promoter of interest.

Agents are screened for biological activity by adding the agent to at least one and usually a plurality of animals. The change in expression of the imaging marker is detected by providing the animal with the appropriate radiolabeled hapten, and performing imaging analysis as described above. The resulting data may then be evaluated by comparison to reference responses, which may include basal readouts in the presence and absence of the agent, responses obtained with other agents, which may or may not include known pharmacologically active compounds, *etc*. Agents of interest for analysis include any biologically active molecule with the capability of modulating, directly or indirectly, the phenotype of interest of a cell of interest.

A plurality of assays may be run in parallel with different agent concentrations to obtain a differential response to the various concentrations. As known in the art, determining the effective concentration of an agent typically uses a range of concentrations resulting from 1:10, or other log scale, dilutions. The concentrations may be further refined with a second series of dilutions, if necessary. Typically, one of these concentrations serves as a negative control, i.e. at zero concentration or below the level of detection of the agent or at or below the concentration of agent that does not give a detectable change in the phenotype. The circulatory flow, interaction with other tissues, and other parameters associated with a physiological response are not found in standard tissue culture formats. Therefore, the resulting assay data is not based on the pattern of drug or toxin exposure that would be found in an animal.

Within living beings, concentration, time and metabolism interact to influence the intensity and duration of a pharmacologic or toxic response. For example, *in vivo* the presence of liver function strongly affects drug metabolism and bioavailability. The development of screening assays that can provide better, faster and more efficient prediction of *in vivo* toxicity and clinical drug performance is of great interest in a number of fields, and is addressed in the present invention.

It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "a vector" includes a plurality of such constructs and reference to "the imaging marker" includes reference to one or more imaging markers and equivalents thereof known to those skilled in the art, and so forth.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

### EXPERIMENTAL

### I. Expression of single chain antibody variable domains on mammalian cells

DNA encoding ScFv FITC-E2 (Vaughan et al., Nat Biotechnol 1996 Mar;14(3):309-14) was isolated by SfiI/NotI digestion of the vector. After blunting the SfiI site with T4 polymerase, this fragment was inserted into the vector CMV+T8 12CA5 KH cut with EcoRI (blunted with T4 polymerase) and NotI. The resulting construct encodes the FITC ScFv as a phosphatidylinositide glycan-linked (GPI) protein containing an amino-terminal 12CA5 epitope and a carboxy-terminal anitbody 179 epitope (A. Lin et al., Science 249, 677-679 (1990); N. Wrighton et al., Science 273, 458-463 (1996); K. Koller et al., Analytical Biochemistry 250, 51-60 (1997)). The mAb 179 epitope is derived from the human placental alkaline phosphatase (HPAP) COOH-terminal sequence. The vector CMV+T8 12CA5 KH contains the CMV promoter, the leader sequence from human CD8, an epitope from the infulenza HA protein recognized by the antibody 12CA5 and the HPAP COOH-terminal sequence. Constructs for ScFv expression as transmembrane proteins were constructed by replacing sequences downstream of the mAb 179 epitope, including the HPAP COOH-terminal signal sequence, with the transmembrane region of the human CD8 antigen (amino acids 153 to 187) fused to either the whole intracytoplasmic domain of the human ζ chain of the T-cell antigen receptor (amino acids 31-142, B. Irving et al., Cell 64, 891-901 (1991)), or a truncated portion of the ζ chain (amino acids 31-52). The truncated version contains the additional residues Ala Gly Glu Leu Ala downstream of ζ chain residue 52. Expression of ScFv FITC in CHO-K1 cells by transient transfection was performed essentially as previously described (Mattheakis et al., Chemistry & Biology **6**, 835-844 (1999)). CHO-K1, 3-5x10⁶ cells were electroporated with 5µg of plasmid DNA in 0.3ml growth medium and subsequently plated on T75 flasks. After a 24 h incubation, cells were harvested and aliquots boiled directly in SDS-PAGE gel loading buffer to make whole cell extracts. Western blotting using mAb 179 was performed as previously described (Northrop et al., Mol. Cell. Biol. 16,2255-2263 (1996)).

### II. Isolation and FACS characterization of ScFv FITC-expressing stable clones

Stable transfectants were isolated essentially as described (Mattheakis et al., 1999). All ScFv FITC expression constructs contain a neo resistance cassette. Primary G418-resistant populations were stained with mAb 179 labeled with fluorescein as follows. Antibody 179 was linked to fluorescein using fluorescein isothiocyanate (FITC, Molecular Probes, Eugene, OR). This modified mAb 179 contained on average six fluoresceins per antibody molecule. Cells, ~3-5x10⁶, were resuspended in 500 µl of PBS, 0.1%BSA containing mAb 179-FITC at a final concentration of 8ug/ml. Cells were rotated in the dark for 45min, spun down, and subsequently resuspended in PBS and enrich sorted using a FACStar plus (Becton-Dickinson, San Jose, CA) based on the highest fluorescence. Enriched populations were cloned by limiting dilution and subsequently screened by ELISA. Briefly, 96well plates of clones were fixed with 200ul per well of ice cold methanol for 10 min. Subsequent steps were done at room temperature (rt). Plates were blocked with PBS, 1%BSA for 30 min and then incubated with mAb 179, 1µg/ml in PBS, 0.1%BSA for Ih. After 3 washes of 10 min each with PBS, wells were incubated with 1µg/ml goat anti-mouse-HRP (Pierce, Rockford, IL) in PBS, 0.1%BSA for 1h, washed with PBS three times as above, developed with a TMB peroxidase substrate colorometric assay (Kirkegaard & Perry Laboratories, Gaithersburg, MD) and the OD₄₁₅ measured. Reactive clones were expanded, stained with mAb 179-fluorescein as above and analyzed using a FACScan (Becton-Dickinson, San Jose, CA) to choose those with the highest expression of the ScFv based on fluorescence. Clones were also analyzed by anti-179 Western blotting to verify the size of the ScFv protein expressed. ScFv-expressing clones were compared to wild-type CHO-K1 cells by FACScan using further reagents as follows in PBS, 0.1%BSA as above for mAb 179-fluorescein staining. FITC-BSA (Sigma, St. Louis, MO) was used at a final concentration of 20 µg/ml and DTPA-FITC and In-DTPA-FITC were each used at a final concentration of 2 µg/ml.

### III. Synthesis of DTPA-FITC and chelation to InCl₃

Diethylenetriaminepenta-acetic acid (DTPA) coupled to fluorescein was synthesized by dropwise addition of an eqimolar amount of DTPA anyhydride (Sigma, St. Louis, MO) in anhydrous DMF to a solution of fluorescein cadaverine (dihydrobromide salt, Molecular Probes, Eugene, OR) containing di-isopropyl ethly amine over a 75 min time period at room temperature. The reaction was allowed to proceed for a further 48 h at rt. The crude mixture of mono and disubstituted DTPA was dried, dissolved in a 2:1 mixture of H₂O:DMF with gentle heating and then cooled to rt at which point the disubstituted product precipitated. The monosubstituted DTPA-FITC was dried and dissolved in 50mM sodium acetate pH 5.1. Chelation of DTPA-FITC to InCl₃ (Aldrich, Milwaukee, WI) was performed in 50mM sodium acetate pH 5.1 using equimolar ratios at rt for 45min. Chelation to ¹¹¹InCl₃ (397.5 Ci/mg, PerkinElmer life sciences, Boston, MA) was also performed in 50mM sodium acetate pH 5.1 at rt, however, the molar ratio of ¹¹¹InCl₃ to DTPA-FITC was ~1:30 and no unlabelled InCl₃ was added. Chelates were diluted in PBS, 0.1% BSA before use in binding experiments.

### IV. Binding of ¹¹¹In-DTPA-FITC to mammalian cell lines in vitro

Wild-type or ScFv FITC-expressing cells were plated at 3x10⁴ cells/well in 96-well plates one day before binding experiments. Cells were washed once with 200ul PBS/well and ¹¹¹In-DTPA-FITC at varying concentrations (maximum of 100nM containing luCi of ¹¹¹In total/well) diluted in 100ul PBS, 0.1% BSA was added. Cells were gently agitated for 45 minutes at rt and then washed three times 250ul/well PBS at 4 degrees C. Individual wells were counted for 1min each in a 1470 Wizard gamma counter (Wallac). For competition experiments, competitor compounds were diluted in 50ul/well of PBS, 0.1 % BSA and added first to washed wells containing cells as above. Tracer consisting of 0.1uCi/well ¹¹¹In-DTPA-FITC at a final concentration of 1nM was then added in a further 50ul/well of the same buffer. Incubations, washes and counting were performed as above.

### V. In Vivo biodistribution studies

DTPA-FITC was chelated as above with ¹¹¹InCl₃. Female 6wk old Balb/c mice were injected via tail vein with a total of 4uCi ¹¹¹In-DTPA-FITC in a volume of 200ul PBS. At various times, the mice were sacrificed and tissue samples taken for weighing and counting. Data was calculated as % injected dose per gram of tissue.

## Claims

1. An non human animal comprising an imaging marker trans gene, wherein said transgene encodes a cell surface polypeptide that binds an imaging radiolabeled hapten at high affinity.

2. The non human animal of Claim 1, wherein said imaging marker transgene encodes a single chain antibody variable region.

3. The non human animal of Claim 2, wherein said single chain antibody variable region specifically binds a small molecule hapten.

4. The non human animal of Claim 3, wherein said hapten comprises a positron-emitting isotope or a single photon-emitting isotope.

5. The non human animal according to Claim 4, wherein said positron-emitting isotope is selected from the group consisting of ¹⁸F; ¹¹C; ¹²⁴I; and ⁷⁶Br, or said photon-emitting isotope is selected from the group consisting of ¹²³I; ¹²⁵I; and ¹³¹I.

6. The non humananimal of Claim 2, wherein said single chain antibody variable region specifically binds a metal chelate.

7. The non human animal of Claim 6, wherein said metal chelate comprises one of ¹¹¹I; ^{99m}Tc; ⁶⁷Ga; or ⁶⁸Ga.

8. The non human animal of Claim 2, wherein said single chain antibody variable region is localized on the cell surface by a glycosylated phosphatidylinositol membrane anchor.

9. The non human animal of Claim 2, wherein said single chain antibody variable region is localized on the cell surface by a transmembrane domain.

10. The non human animal of Claim 2, wherein expression of said single chain antibody variable region is controlled by a constitutive promoter.

11. The non-human animal of Claim 2, wherein expression of said single chain antibody variable region is controlled by a regulated promoter.

12. The non-human animal of Claim 2, wherein essentially all the cells of said animal comprise said transgene.

13. The non-human animal of Claim 2, wherein a subset of cells in said animal comprise said transgene.

14. The non-human animal of Claim 13, wherein said subset of cells are transplanted into said animal.

15. A non-invasive method of detecting gene expression *in vivo*, the method comprising:
- localising expression of an imaging marker transgene in a non-human animal through PET or SPECT acquisition and reconstruction
the animal comprising an imaging marker transgene, wherein said transgene encodes a cell surface polypeptide that binds a radiolabelled hapten at high affinity, and the animal being an animal to which an imaging radiolabelled hapten has been administered.

16. The method according to Claim 15, wherein said imaging marker transgene encodes a single chain antibody variable region.

17. The method according to Claim 16, wherein said single chain antibody variable region specifically binds a small molecule hapten.

18. The method according to Claim 17, wherein said hapten comprises a positron-emitting isotope or a single photon-emitting isotope.

19. The method according to Claim 18, wherein said positron-emitting isotope is selected from the group consisting of ¹⁸F; ¹¹C; ¹²⁴I; and ⁷⁶Br, or said photon-emitting isotope is selected from the group consisting of ¹²³I; ¹²⁵I; and ¹³¹I.

20. The method according to Claim 16, wherein said single chain antibody variable region specifically binds a metal chelate.

21. The method according to Claim 20, wherein said metal chelate comprises one of ¹¹¹I; ^{99m}Tc; ⁶⁷Ga; or ⁶⁸Ga.

22. The method according to Claim 16, wherein said non-human animal is an animal to which a biologically active molecule has been administered.

23. The method according to Claim 16, wherein said single chain antibody variable region is localized on the cell surface by a glycosylated phosphatidylinositol membrane anchor.

24. The method according to Claim 16, wherein said single chain antibody variable region is localized on the cell surface by a transmembrane domain.

25. The method according to Claim 16, wherein expression of said single chain antibody variable region is controlled by a constitutive promoter.

26. The method according to Claim 16, wherein expression of said single chain antibody variable region is controlled by a regulated promoter.

27. The method according to Claim 16, wherein essentially all the cells of said non-human animal comprise said transgene.

28. The method according to Claim 16, wherein a subset of cells in said non human animal comprise said transgene.

29. The method according to Claim 28, wherein said non-human animal is an animal to which said subset of cells has been transplanted.

## Patentansprüche

1. Nicht-humanes Tier, das ein bildgebendes Marker-Transgen umfaßt, worin das Transgen ein Zelloberflächenpolypeptid codiert, das ein bildgebendes radiomarkiertes Hapten mit hoher Affinität bindet.

2. Nicht-humanes Tier gemäß Anspruch 1, worin das bildgebende Marker-Transgen eine variable Region eines einkettigen Antikörpers codiert.

3. Nicht-humanes Tier gemäß Anspruch 2, worin die variable Region des einkettigen Antikörpers spezifisch ein kleines Molekül als Hapten bindet.

4. Nicht-humanes Tier gemäß Anspruch 3, worin das Hapten ein Positronen emittierendes Isotop oder ein Einzelphotonen emittierendes Isotop ist.

5. Nicht-humanes Tier gemäß Anspruch 4, worin das Positronen emittierende Isotop aus der Gruppe ausgewählt ist, die aus ¹⁸F, ¹¹C, ¹²⁴I und ⁷⁶Br besteht, oder das Photonen emittierende Isotop aus der Gruppe ausgewählt ist, die aus ¹²³I, ¹²⁵I und ¹³¹I besteht.

6. Nicht-humanes Tier gemäß Anspruch 2, worin die variable Region des einkettigen Antikörpers spezifisch ein Metallchelat bindet.

7. Nicht-humanes Tier gemäß Anspruch 6, worin das Metallchelat eines aus ¹¹¹I, ^{99m}Tc, ⁶⁷Ga oder ⁶⁸Ga umfaßt.

8. Nicht-humanes Tier gemäß Anspruch 2, worin die variable Region des einkettigen Antikörpers auf der Zelloberfläche durch einen glycosylierten Phosphatidylinosit-Membrananker lokalisiert ist.

9. Nicht-humanes Tier gemäß Anspruch 2, worin die variable Region des einkettigen Antikörpers auf der Zelloberfläche durch eine Transmembrandomäne lokalisiert ist.

10. Nicht-humanes Tier gemäß Anspruch 2, worin die Expression der variablen Region des einkettigen Antikörpers durch einen konstitutiven Promotor kontrolliert wird.

11. Nicht-humanes Tier gemäß Anspruch 2, worin die Expression der variablen Region des einkettigen Antikörpers durch einen regulierten Promotor kontrolliert wird.

12. Nicht-humanes Tier gemäß Anspruch 2, worin im wesentlichen alle Zellen des Tieres das Transgen umfassen.

13. Nicht-humanes Tier gemäß Anspruch 2, worin eine Untergruppe von Zellen in dem Tier das Transgen umfassen.

14. Nicht-humanes Tier gemäß Anspruch 13, worin die Untergruppe von Zellen in das Tier transplantiert sind.

15. Nicht-invasives Verfahren zur Detektion der Genexpression in vivo, wobei das Verfahren das Lokalisieren der Expression eines bildgebenden Marker-Transgens in einem nicht-humanen Tier durch PET- oder SPECT-Erfassung und -Rekonstruktion umfaßt, wobei das Tier ein bildgebendes Marker-Transgen umfaßt, worin das Transgen ein Zelloberflächenpolypeptid codiert, das ein radiomarkiertes Hapten mit hoher Affinität bindet, und wobei das Tier ein Tier ist, an das ein bildgebendes radiomarkiertes Hapten verabreicht wurde.

16. Verfahren gemäß Anspruch 15, worin das bildgebende Marker-Transgen eine variable Region eines einkettigen Antikörpers codiert.

17. Verfahren gemäß Anspruch 16, worin die variable Region des einkettigen Antikörpers spezifisch ein kleines Molekül als Hapten bindet.

18. Verfahren gemäß Anspruch 17, worin das Hapten ein Positronen emittierendes Isotop oder ein Einzelphotonen emittierendes Isotop umfaßt.

19. Verfahren gemäß Anspruch 18, worin das Positronen emittierende Isotop aus der Gruppe ausgewählt ist, die aus ¹⁸F, ¹¹C, ¹²⁴I und ⁷⁶Br besteht, oder das Photonen emittierende Isotop aus der Gruppe ausgewählt ist, die aus ¹²³I, ¹²⁵I und ¹³¹I besteht.

20. Verfahren gemäß Anspruch 16, worin die variable Region des einkettigen Antikörpers spezifisch ein Metallchelat bindet.

21. Verfahren gemäß Anspruch 20, worin das Metallchelat eines aus ¹¹¹I, ^{99m}Tc, ⁶⁷Ga oder ⁶⁸Ga umfaßt.

22. Verfahren gemäß Anspruch 16, worin das nicht-humane Tier ein Tier ist, an das ein biologisch aktives Molekül verabreicht wurde.

23. Verfahren gemäß Anspruch 16, worin die variable Region des einkettigen Antikörpers auf der Zelloberfläche durch einen glycosylierten Phosphatidylinosit-Membrananker lokalisiert ist.

24. Verfahren gemäß Anspruch 16, worin die variable Region des einkettigen Antikörpers auf der Zelloberfläche durch eine Transmembrandomäne lokalisiert ist.

25. Verfahren gemäß Anspruch 16, worin die Expression der variablen Region des einkettigen Antikörpers durch einen konstitutiven Promotor kontrolliert wird.

26. Verfahren gemäß Anspruch 16, worin die Expression der variablen Region des einkettigen Antikörpers durch einen regulierten Promotor kontrolliert wird.

27. Verfahren gemäß Anspruch 16, worin im wesentlichen alle Zellen des nicht-humanen Tieres das Transgen umfassen.

28. Verfahren gemäß Anspruch 16, worin eine Untergruppe von Zellen in dem Tier das Transgen umfassen.

29. Verfahren gemäß Anspruch 28, worin das nicht-humane Tier ein Tier ist, in das die Untergruppe von Zellen transplantiert wurde.

## Revendications

1. Animal non humain comprenant un transgène marqueur d'imagerie, dans lequel ledit transgène code un polypeptide de surface cellulaire qui se lie à un haptène radiomarqué d'imagerie avec une affinité élevée.

2. Animal non humain selon la revendication 1, dans lequel ledit transgène marqueur d'imagerie code une région variable d'anticorps à chaîne unique.

3. Animal non humain selon la revendication 2, dans lequel ladite région variable d'anticorps à chaîne unique se lie spécifiquement à une petite molécule haptène.

4. Animal non humain selon la revendication 3, dans lequel ledit haptène comprend un isotope émetteur de positron ou un isotope émetteur de photon unique.

5. Animal non humain selon la revendication 4, dans lequel ledit isotope émetteur de positron est choisi dans le groupe comprenant ¹⁸F ; ¹¹C ; ¹²⁴I ; et ⁷⁶Br, ou ledit isotope émetteur de photon est choisi dans le groupe comprenant ¹²³I ; ¹²⁵I ; et ¹³¹I.

6. Animal non humain selon la revendication 2, dans lequel ladite région variable d'anticorps à chaîne unique se lie spécifiquement à un chélate métallique.

7. Animal non humain selon la revendication 6, dans lequel le chélate métallique comprend l'un de ¹¹¹I ; ^{99m}Tc ; ⁶⁷Ga ; ou ⁶⁸Ga.

8. Animal non humain selon la revendication 2, dans lequel ladite région variable d'anticorps à chaîne unique est localisée sur la surface cellulaire par un ancrage de membrane phosphatidylinositol glycosylé.

9. Animal non humain selon la revendication 2, dans lequel ladite région variable d'anticorps à chaîne unique est localisée sur la surface cellulaire par un domaine transmembranaire.

10. Animal non humain selon la revendication 2, dans lequel l'expression d'une région variable d'anticorps à chaîne unique est contrôlée par un promoteur constitutif.

11. Animal non humain selon la revendication 2, dans lequel l'expression de ladite région variable d'anticorps à chaîne unique est contrôlée par un promoteur régulé.

12. Animal non humain selon la revendication 2, dans lequel sensiblement toutes les cellules dudit animal comprennent ledit transgène.

13. Animal non humain selon la revendication 2, dans lequel un sous-ensemble de cellules dans ledit animal comprennent ledit transgène.

14. Animal non humain selon la revendication 13, dans lequel ledit sous-ensemble de cellules est transplanté dans ledit animal.

15. Procédé non invasif pour détecter l'expression d'un gène *in vivo,* le procédé comprenant :
- la localisation de l'expression d'un transgène marqueur d'imagerie dans un animal non humain par acquisition et reconstruction PET ou SPECT
l'animal comprenant un transgène de marqueur d'imagerie, ledit transgène codant un polypeptide de surface cellulaire se liant à un haptène radiomarqué avec une affinité élevée, et l'animal étant un animal auquel un haptène radiomarqué d'imagerie a été administré.

16. Procédé selon la revendication 15, dans lequel ledit transgène marqueur d'imagerie code une région variable d'anticorps à chaîne unique.

17. Procédé selon la revendication 16, dans lequel ladite région variable d'anticorps à chaîne unique se lie spécifiquement à une petite molécule haptène.

18. Procédé selon la revendication 17, dans lequel ledit haptène comprend un isotope émetteur de positron ou un isotope émetteur de photon unique.

19. Procédé selon la revendication 18, dans lequel ledit isotope émetteur de positron est choisi dans le groupe comprenant ¹⁸F ; ¹¹C ; ¹²⁴I ; et ⁷⁶Br, ou ledit isotope émetteur de photon est choisi dans le groupe comprenant ¹²³I ; ¹²⁵I ; et ¹³¹I.

20. Procédé selon la revendication 16, dans lequel ladite région variable d'anticorps à une seule chaîne se lie spécifiquement à un chélate métallique.

21. Procédé selon la revendication 20, dans lequel ledit chélate métallique comprend l'un de ¹¹¹I ; ^{99m}Tc ; ⁶⁷Ga ; ou ⁶⁸Ga.

22. Procédé selon la revendication 16, dans lequel ledit animal non humain est un animal auquel une molécule biologiquement active a été administrée.

23. Procédé selon la revendication 16, dans lequel ladite région variable d'anticorps à chaîne unique est localisée sur la surface cellulaire par un ancrage membranaire phosphatidylinositol glycosylé.

24. Procédé selon la revendication 16, dans lequel ladite région variable d'anticorps à chaîne unique est localisée sur la surface cellulaire par un domaine transmembranaire.

25. Procédé selon la revendication 16, dans lequel l'expression de ladite région variable d'anticorps à chaîne unique est contrôlée par un promoteur constitutif.

26. Procédé selon la revendication 16, dans lequel l'expression de ladite région variable d'anticorps à chaîne unique est contrôlée par un promoteur régulé.

27. Procédé selon la revendication 16, dans lequel sensiblement toutes les cellules dudit animal non humain comprennent ledit transgène.

28. Procédé selon la revendication 16, lequel un sous-ensemble de cellules dans ledit animal comprennent ledit transgène.

29. Procédé selon la revendication 28, dans lequel ledit animal non humain est un animal auquel ledit sous-ensemble de cellules a été transplanté.
